Europäisches Patentamt

European Patent Office

Office européen des brevets

(19)

(11) Numéro de publication : **0 515 456 B1**

## FASCICULE DE BREVET EUROPEEN

(12)

(45) Date de publication du fascicule du brevet :
04.05.94 Bulletin 94/18

(51) Int. Cl.[5] : **C07H 3/06,** A23L 1/30,
A23L 1/236, C08B 37/18,
C12P 19/18

(21) Numéro de dépôt : 91903932.1

(22) Date de dépôt : 22.02.91

(86) Numéro de dépôt international :
PCT/BE91/00014

(87) Numéro de publication internationale :
WO 91/13076 05.09.91 Gazette 91/21

(54) FRUCTO-OLIGOSACCHARIDES RAMIFIES, PROCEDE POUR LEUR OBTENTION ET UTILISATION DES PRODUITS LES CONTENANT.

(30) Priorité : 23.02.90 BE 9000213

(43) Date de publication de la demande :
02.12.92 Bulletin 92/49

(45) Mention de la délivrance du brevet :
04.05.94 Bulletin 94/18

(84) Etats contractants désignés :
AT BE CH DE DK ES FR GB GR IT LI LU NL SE

(56) Documents cités :
EP-A- 0 337 889
Liebigs Annalen der Chemie, Verlag Chemie
(Weinheim, DE), vol. 614, 1958, HH Schlubach
et al.: "Die Bildung der verzweigten Polyfructosane in den Roggenhalmen", pages
126-136, see pages 131-2, 134
Patent Abstracts of Japan, vol. 13, No.151
(C-584)(3499) 12 April 1989

(56) Documents cités :
Journal of the Chemical Society, 1951, part II,
The Chemical Society, (London, GB), P C Arni
et al.: "Studies on Fructosans", pages
1822-30

(73) Titulaire : RAFFINERIE TIRLEMONTOISE
Avenue de Tervuren 182
B-1150 Bruxelles (BE)

(72) Inventeur : COUSSEMENT, Paul
Ganzendries 63
B-3212 Pellenberg (BE)
Inventeur : DE LEENHEER, Leen
Jezus Eiklaan 64
B-3080 Tervuren (BE)
Inventeur : SMITS, Georges
Dr. De Cockstraat 16
B-9308 Gijzegem-Aalst (BE)

(74) Mandataire : Van Malderen, Michel et al
Office van Malderen Place Reine Fabiola 6/1
B-1080 Bruxelles (BE)

## Description

### Objet de l'invention.

La présente invention concerne des fructo-oligosaccharides ramifiés présentant notamment des propriétés édulcorantes et d'agents de masse.

Elle concerne également les produits contenant ces fructo-oligosaccharides ramifiés et leur utilisation.

La présente invention s'étend enfin aux procédés particuliers pour leur préparation.

### Résumé de l'état de la technique.

Un mode de vie sédentaire où le travail physique a presque disparu a considérablement modifié les besoins alimentaires.

En règle générale, on mange trop et souvent de manière déséquilibrée. Trop de graisses, trop de sel trop peu de sucres dits lents (complexes), et trop peu de fibres.

En particulier, la proportion de sucres dits lents a considérablement diminué dans nos régimes alimentaires, limitant ainsi leur apport énergétique.

Actuellement, les sucres dits rapides et les graisses fournissent les principaux apports énergétiques dans notre alimentation. On sait depuis longtemps, qu'une alimentation déséquilibrée conduit à long terme à des carences graves ou même à des maladies par exemple cardio-vasculaires.

En outre, une alimentation pauvre en fibres semble être la cause de nombreuses atteintes du système digestif.

On a de plus en plus conscience du lien étroit qui existe entre l'état de santé général et la manière de se nourrir. Aussi tend-on à adapter l'alimentation ou plus particulièrement la diététique, à nos besoins individuels.

Entre autres, le développement et l'utilisation d'édulcorants à haut pouvoir sucrant ont permis au consommateur de mieux adapter son alimentation à ses besoins. Ces édulcorants permettent de donner un goût sucré aux aliments qui les contiennent sans devoir faire appel à des sucres fortement caloriques.

De cette manière, des boissons d'un apport calorique négligeable sont, par exemple, apparues sur le marché.

D'autre part, l'utilisation d'agents de masse est souvent indispensable dans une série d'aliments tels que la pâtisserie, la confiserie...

Habituellement on utilise du saccharose, des sirops de glucose ou de fructose comme agents de masse. Néanmoins ces sucres sont fortement caloriques.

A nouveau, il est apparu sur le marché des "sucres de remplacement" qui sont caractérisés par un apport calorique moindre.

Plus particulièrement, l'agent de masse idéal serait un produit naturel peu ou pas calorique, d'emploi sûr, sain, bien toléré par l'organisme, sans goût particulier et d'une mise en oeuvre comparable à celle du saccharose.

Les agents de masse répondant à ces exigences ont un grand potentiel commercial.

Les agents de masse sont faiblement caloriques s'ils satisfont à l'une des deux conditions suivantes :
- n'être que partiellement ou pas du tout assimilés dans le sang via l'intestin grêle;
- être assimilés dans le sang par l'intestin grêle mais peu ou pas métabolisés par l'organisme.

L'utilisation des fructo-oligosaccharides linéaires comme agents de masse est connue. Ces sucres, dont la présence naturelle dans l'alimentation est connue depuis longtemps, peuvent être obtenus par voie enzymatique (voir Albon et Al., J. Chem. Soc. (1953), p. 24-27; Kawai et Al., Agr. Biol. Chem. Vol. 37, n°9 (1973, p. 2111-2119; Tomoda et Al., Kyoritsu Yakka Daigaku Nempo vol. 20 (1975) p. 1-8) ou par hydrolyse de l'inuline.

Les fructo-oligosaccharides des linéairs peuvent être produits industriellement à partir de sucre comme décrit dans le document GB-A-2000144 en obtenant des produits du type $GF_n$ (G = Glucose, F = Fructose, n = 2 à 5, liaisons du type $\beta$ (1-2), ou par hydrolyse de l'inuline , produisant un mélange de $GF_n$ et $F_n$ (n = 2 à 10 et plus).

Ces fructo-oligosaccharides présentent les propriétés avantageuses suivantes : goût neutre et sucré, absence d'odeur, faible valeur calorique, effet de fibres alimentaires, effet bifidogène, caractère naturel et non toxique. De plus, ces produits présentent des caractéristiques technologiques telles qu'ils peuvent être mis en oeuvre selon les modes de préparation classiques des sucres et des sirops.

De même, ils peuvent être utilisés, comme sucre de remplacement dans les applications classiques, par exemple comme édulcorants dans les aliments et les boissons ou comme matières de base ou excipients pour la préparation de produits pharmaceutiques.

De plus, ils peuvent être utilisés dans toutes les applications industrielles prévues pour les sucres et les sirops telles que la production d'adhésifs, d'humidificateurs, d'insecticides, de colorants, d'agents de tannage, d'isolateurs électriques, de liants pour noyaux de fonderie, ou de manière plus générale, comme matière ramollissante et/ou épaississante et, bien entendu, dans le domaine plus particulier de l'alimentation comme produit de faible valeur calorique ou comme produit à effet de fibres alimentaires ou à effet bifidogène.

En règle générale, les agents de masse peu caloriques ne sont que partiellement assimilés dans l'intestin grêle et, de ce fait, passent dans le gros intestin où ils subissent en partie ou totalement une fermentation due à la flore intestinale.

Il s'agit d'un processus naturel que subissent toutes les fibres et qui est la cause de l'effet bénéfique dû aux fibres.

L'agent de masse est transformé par fermentation dans le gros intestin en une série de produits de réaction dont les principaux sont des acides gras volatils (VFA = Volatile Fatty Acids) et des gaz tels que $CO_2$, $H_2$ et dans certains cas $CH_4$.

Ces gaz sont principalement éliminés par le sang et les poumons ou, en partie, par flatulence.

Lors du passage de l'agent de masse dans l'intestin grêle, les molécules de celui-ci absorbent de l'eau et passent dans le gros intestin. Ceci peut provoquer des diarrhées osmotiques lorsque l'agent de masse est pris en grande quantité. Bien entendu, cet effet dépend, essentiellement du pouvoir absorbant en eau de l'agent de masse et est, en général, plus élevé dans le cas de substances essentiellement à base de monosaccharides, que dans le cas des saccharides à degré de polymérisation plus élevé.

En particulier, les fructo-oligosaccharides linéaires qui ne sont pas ou peu assimilés dans l'estomac et l'intestin grêle, se retrouvent presque entièrement dans le gros intestin et, du fait de leur nature oligosaccharidique (faible degré de polymérisation), ils ne provoquent de diarrhées osmotique, que lorqu'ils sont pris en grande quantité.

Cependant, les fructo-oligosaccharides linéaires, lorsqu'ils sont ingérés en grande quantité, peuvent provoquer une série d'inconvénients (et de malaises) dus principalement à leur caractère fermentable : flatulence augmentée, crampes intestinales, bruits gastriques, selles molles ou même diarrhées.

Plus précisément, on ne trouve pas de traces de fructo-oligosaccharides linéaires dans les selles, même pour de grandes quantités ingérées : ceci démontre qu'ils ont été complètement soumis à la fermentation par la flore intestinale.

En fait, il a été prouvé que les fructo-oligosaccharides linéaires sont dégradés par les bactéries appartenant à la famille des bifidobactéries, ce qui met en évidence le caractère bifidogène des fructo-oligosaccharides linéaires.

Il est bien entendu que ces divers phénomènes dépendent de la dose ingurgitée, de la sensibilité de la personne concernée, de la forme sous laquelle on donne le produit contenant les fructo-oligosaccharides, de l'étalement dans le temps des prises ingérées, éventuellement de la période d'adaptation au produit et de la composition des autres ingrédients et, enfin, de la nature de l'agent de masse ingéré.

Ce sont d'ailleurs des malaises connus depuis longtemps, en particulier lors de l'ingestion de grandes quantités de prunes, de cerises ou d'oignons.

Ces effets secondaires indésirables sont la cause du fait que l'utilisation de ces agents de masse faiblement caloriques n'est pas illimitée.

En particulier, pour certains agents, le législateur a défini une dose journalière maximale. Cette dose s'élève par exemple, à 30 g pour certains sucres-alcools (polyalcools dérivés du sucre).

Même dans le cas d'agents de masse constitués de fructo-oligosaccharides linéaires, ces inconvénients subsistent bien que ces derniers soient en général mieux tolérés par l'organisme probablement du fait que l'effet osmotique est moindre que pour certains sucres-alcools.

Plus particulièrement, il est apparu que les fructo-saccharides à longue chaîne comme l'inuline, provoquent des phénomènes osmotiques encore plus réduits. Cependant, les effets négatifs dus à la fermentation par la flore intestinale n'ont pas totalement disparu.

De plus, ces fructo-oligosaccharides présentent une solubilité d'autant plus faible et une viscosité d'autant plus élevée que la longueur de chaîne est grande.

Il en résulte que les fructo-saccharides à longue chaîne ne présentent pas toutes les propriétés technologiques adéquates pour être utilisés comme agent de masse en lieu et place du sucre.

D'autre part, des polymères du fructose ont déjà été décrits dans les documents suivants:

- Dans Indian Journal of Biochemistry & Biophysics, vol. 13, Dec. 1976, pp. 398-410, Satyanarayana décrit des oligosaccharides ramifiés que l'on peut trouver en petites quantités dans la nature. Il semble que la fructosyltransférase qui a été isolée à partir de l'Agave Vera Cruz ne soit pas à même de synthétiser cet oligosaccharide ramifié; de cette manière, il n'est pas possible de l'obtenir en grandes quantités.
- De même, dans Liebigs Annalen der Chemie, 614, 126 (1958), schlubach a décrit des oligosaccharides ramifiés que l'on peut trouver dans la nature en faibles quantités.

- Dans Liebigs Annalen der Chemie, 635, 154 (1959), Schlubach a décrit également des produits naturels constitués d'unités de fructose polymérisés et ramifiés que l'on ne peut obtenir naturellement en grandes quantités.
- Dans Agric. Biol. Chem., 52(5) 1303-1304 (1988), Muramatsu décrit l'obtention par un microorganisme à partir de sucrose, des oligosaccharides dont la structure est identique aux produits décrits dans le brevet EP-0307158.
- Dans Carbohydrate Research, 180 (1988), 315-324, Brasch et al. ont décrit un polymère de fructose (longueur de chaîne moyenne: 18) où 15% des unités de fructose comportent une ramification sur la position 0-6. Néanmoins, du fait de leur trop grande longueur de chaîne, ces molécules ne constituent pas un agent de masse adéquat.
- Le brevet EP-0 307 158 de Nihon Shokuhin décrit un polymère de fructose où apparaît une ramification sur une unité de glucose.

Le document J. of Chem. Soc., p.1822-1830, (1951) décrit un polyfructose ramifié dont le nombre d'unités de fructose est estimé entre 18 et 30 unités.

Le document Liebigs Annalen der Chemie, 647, 41 (1961) décrit des fructo-oligosaccharides présentant soit une seule ramification sur l'unité de glucose (le néokestose et la série du néobifurcose), soit une seule ramification sur une unité de fructose (le kestose, le bifurcose et les dérivés phlein du bifurcose).

Le document New Zealand J. of Technologie, vol.1, N° 1, p.27-31 (1985) décrit un fructo-oligosaccharide ramifié extrait du "cordyline australis" avec une longueur moyenne de chaîne de 18 unités.

La demande de brevet GB-2 105 338 décrit un procédé de traitement enzymatique permettant d'obtenir un oligosaccharide non ramifié constitué d'une à quatre molécules de fructose liée(s) à une molécule de sucrose.

## Buts de l'invention

La présente invention vise à produire des agents de masse ne présentant pas ou de manière fortement atténuée les troubles digestifs indisérables des agents de masse conventionnels.

Un autre but de la présente invention est de fournir un agent de masse présentant les mêmes propriétés avantageuses que les fructo-oligosaccharides linéaires, telles que goût neutre et sucré, absence d'odeur, faible valeur calorique, effet de fibres alimentaires, effet bifidogène caractère naturel et non-toxique du produit.

Un autre but de la présente invention consiste à obtenir des produits plus solubles.

Un autre but de la présente invention consiste à fournir des produits qui peuvent être mis en oeuvre de manière conventionnelle, selon les procédés classiques de préparation des sucres et des sirops.

D'autres buts complémentaires de la présente invention résident dans l'utilisation du produit de l'invention comme édulcorant, comme aliment faiblement calorique ou faiblement cariogène, comme produit bifidogène ou à effet de fibres, comme moyen d'abaisser le taux de cholestérol ou d'améliorer l'état de la flore intestinale,...

D'autres buts et avantages apparaissent dans la description qui suit.

## Description de l'invention

La présente invention concerne un fructooligosaccharide ramifié composé d'une chaîne principale et d'une ou plusieurs chaînes latérales caractérisé en ce que la chaîne principale et la ou les chaînes latérales sont constituées, en majorité d'unités de fructose et comportent de préférence de 1 à 15 unités; la chaîne principale comporte de 2 à 15 unités et au moins une ramification sur une unité de fructose, à l'exclusion des produits naturels: kestose, O-$\beta$-D-fructofuranosyl-(2$\rightarrow$1)-O-[O-$\beta$-D-fructofuranosyl-(2$\rightarrow$6)]-$\beta$-D-fructofuranosyl-$\alpha$-D-glucopyranoside, O-$\beta$-D-fructofuranosyl-(2$\rightarrow$1)-$\beta$-D-fructofuranosyl-(2$\rightarrow$1)-O-[O-$\beta$-D-fructofuranosyl-(2$\rightarrow$6)]-$\beta$-D-fructofuranosyl-$\alpha$-D-glucopyranoside et des produits ayant la structure suivante:

$$X \underset{\phantom{a}}{\overset{\phantom{a}}{\longrightarrow}} \left[ \begin{array}{c} 2Fru6 \quad - \quad 2\ Fru6 \\ \quad | \\ \quad 1 \\ \quad | \\ \quad | \\ 2Fru \end{array} \right] \quad Y$$

$n$

a)  $X = Glu1$  $n = 2$  $Y = 2Fru$

b)  $X = Glu1$  $n = 3$  $Y = 2Fru6-2Fru$

c)  $X = Fru1$  $n = 3$  $Y = 2Fru6-2Fru$

d)  $X = Glu1$  $n = 3$  $Y = 2Fru$

e)  $X = Glu1-2Fru6$  $n = 2$

f)  $X = Glu1$  $n = 1$  $Y = -(2Fru6)-_m$

$$m = 1 \text{ à } 12$$

De préférence, la chaîne principale est constituée d'unités de fructose.

Avantageusement, la ou les chaînes latérales sont constituées d'unités de fructose.

Selon une forme d'exécution préférée de l'invention, la ou les chaînes latérales ne sont pas ramifiées.

Selon une autre forme d'exécution préférée de l'invention, une ou plusieurs chaînes latérales sont à leur tour ramifiées en des chaînes latérales supplémentaires.

La présente invention concerne également une composition constituée d'un ou plusieurs fructooligosaccharides ramifiés selon l'invention, et plus particulièrement, des mélanges comportant outre le ou les fructo-oligosaccharides ramifiés d'autres ingrédients tels que des protéines, des lipides ou acides gras, des hydrates de carbone, des fibres et d'autres additifs.

En particulier, ces ingrédients peuvent être des produits repris dans la liste non limitative suivante: des édulcorants tels que le sucrose, les produits d'hydrolyse de l'amidon, la palatinose, le saccharose, le glucose, le fructose, les sirops de glucose ou les polyalcools dérivés de sucre tels que le sorbitol, le xylitol, l'érythritol, le mannitol, le maltitol, le lactitol, l'isomalt, le leucritol; des agents de masse tels que le polydextrose, la cellulose, l'hémicellulose, les fructo-oligosaccharides; ou encore des édulcorants à haut pouvoir sucrant tels que l'aspartame, l'acésulfame, la saccharine, la stévioside, le sucralose et autres édulcorants dipeptidiques.

Les produits selon l'invention sont particulièrement adéquats pour être utilisés dans l'alimentation humaine ou animale, comme agents de masse, comme édulcorants, comme aliments faiblement caloriques, ou faiblement cariogènes, comme produits bifidogènes ou améliorant la flore intestinale, comme produits à effet de fibres alimentaires, comme agents diminuant le taux de cholestérol ou encore pour améliorer la tolérance des produits alimentaires. L'invention concerne également une composition pharmaceutique comprenant un fructo-oligosaccharide ramifié selon l'invention et éventuellement un support.

Enfin, la présente invention concerne également les procédés de préparation de ces fructo-oligosaccharides ramifiés.

En particulier, les produits selon l'invention peuvent être obtenus par synthèse à partir de sucre ou de mélanges de fructose, éventuellement par l'intermédiaire de catalyseurs ou d'enzymes ou par interaction d'enzymes différentes sur la saccharose des fructo-oligosaccharides, inuline ou fructanes ou par voie chimique, c'est-à-dire par polymérisation principalement du fructose, ou par extraction à partir de sources végétales contenant lesdits fructanes, ou encore par hydrolyse des polymères de fructose ramifiés.

Les fructo-oligosaccharides selon l'invention peuvent, en particulier, être obtenus à partir du saccharose, de sirops de fructose, de fructose sous forme cristalline, de fructanes, de levanes, d'inuline, de leurs produits d'hydrolyse ou, enfin, de produits extraits de plantes contenant des fructanes.

Par l'intervention de plusieurs enzymes, comme par exemple le fructosyltransférase, le levansucrase,..., il est également possible de former de façon sélective des liaisons permettant l'implantation d'unités de fructose à des positions déterminées sur d'autres unités de fructose dans les chaînes oligosaccharidiques. On peut utiliser les enzymes, soit consécutivement, soit simultanément. De préférence, on utilisera des enzymes qui forment préférentiellement des liaisons différentes à celles du substrat.

Pour la production de fructo-oligosaccharides ramifiés, on peut utiliser des procédés de condensation ou de synthèse, enzymatiques ou non, ou encore des procédés hydrolytiques.

Par condensation de monomères de fructose, on peut obtenir une polymérisation "at random", qui peut être suivie d'une séparation chromatographique.

On peut également obtenir des fructo-oligosaccharides ramifiés par hydrolyse de molécules ramifiées synthétisées ou existant à l'état naturel.

Dans ce cas, on peut également utiliser plusieurs techniques différentes d'hydrolyse, notamment la technique d'hydrolyse acide, la technique d'hydrolyse alcaline ou la technique d'hydrolyse enzymatique.

En particulier, l'hydrolyse enzymatique spécifique comme par exemple l'interaction de l'endoinulase sur le mélange de fructanes ramifiés, isolés du Cordyline australis ("cabbage tree") qui attaque spécifiquement les liaisons $\beta(2\rightarrow1)$, donne lieu à des fructo-oligosaccharides ramifiés.

Le résultat dépend de la nature du matériau de départ et de la technique d'hydrolyse utilisée et, éventuellement, des enzymes utilisées pour le procédé d'hydrolyse et des conditions de réaction. Selon les conditions de réaction, de nouvelles ramifications peuvent se produire pendant la réaction d'hydrolyse des molécules ramifiées ou linéaires.

Les groupes terminaux réducteurs peuvent en plus être transformés chimiquement, comme par exemple, par oxydation, par réduction ou par hydrogénation,...

La présente invention concerne également un procédé permettant de rendre l'inuline plus soluble par une hydrolyse très partielle, suivie d'une réaction de ramification.

Les procédés utilisés et les produits obtenus sont mieux décrits à l'aide des exemples qui suivent.

## Exemple 1: Obtention de polymères de fructane ramifiés par hydrolyse

Un mélange de fructanes ramifiés peut être obtenu à partir du "cabbage tree" (Cordyline australis) par extraction au méthanol, lavage à l'eau suivi d'une précipitation dans une solution d'acétone/éthanol et séchage.

Les substances obtenus sont principalement constituées d'unités de glucose et de fructose dans un rapport de 1 à 16, disposées en chaînes d'un degré de polymérisation moyen égal à 18, où environ 15% des unités de fructose présentent une ramification.

Une solution à 25° Brix est chauffée jusqu'à 90°C, puis du HCl 2N est ajouté rapidement jusqu'à obtention d'un pH de 2,5. Après 2 minutes de réaction, l'hydrolyse est arrêtée en ajoutant du NaOH (3 M) jusqu'à obtention d'un pH de 6,5.

Cette solution est ensuite filtrée et déminéralisée par un double traitement sur des échangeurs d'ions cationiques et anioniques. Le glucose, le fructose et le saccharose formés par hydrolyse sont ensuite éliminés sur une colonne cationique sous forme potassium par séparation chromatographique. Ainsi, le mélange d'oligosaccharides obtenu contient entre 5 et 10 % de mono- et disaccharides et de 90 à 95 % d'oligosaccharirides. Le degré de polymérisation moyen de la composition ainsi obtenue est d'environ 5 et environ 40 % des molécules y sont ramifiées.

## EXEMPLE 2 ; Obtention du produit selon l'invention par synthèse

Dans ce cas, on utilise l'enzyme levansucrase dérivée du Bacillus subtilis.

On a remarqué que cette enzyme, par incubation dans des conditions appropriées en présence de saccharose et de fructo-oligosaccharides du type $\beta(2-1)$ forme des fructo-oligosaccharides ramifiés.

Dans cet exemple, les fructo-oligosaccharides de base utilisés sont obtenus par hydrolyse enzymatique de l'inuline par l'endo-inulinase, obtenu à partir de la préparation enzymatique Novozyme 230.

Une solution à 40° Brix constituée de 50 % de saccharose et de 50 % de fructo-oligosaccharides de base est préparée dans une solution tampon au phosphate à 0,05 M à un pH de 6.

On ajoute au mélange une solution de levansucrase qui contient 20 U/ml - 1 U étant la quantité d'enzyme nécessaire à la libération de 1 micromôle de glucose par minute - de telle manière à obtenir 4 U de levansucrase par gramme de matière sèche d'hydrate de carbone dans le mélange. La solution est ensuite incubée à 30°C pendant 18 heures dans un agitateur orbital (150 t/min) Ensuite, la réaction enzymatique est arrêtée par la cuisson du mélange de réaction pendant 5 minutes. Des $\beta$-Fructanes linéaires à longue chaîne (DP>12) sont séparés par précipitation avec 80 % d'éthanol.

Après centrifugation (30 minutes à 5000 t/min) et évaporation de l'éthanol sous vide, la solution est déminéralisée sur des échangeurs d'ions tels que décrit dans l'exemple 1.

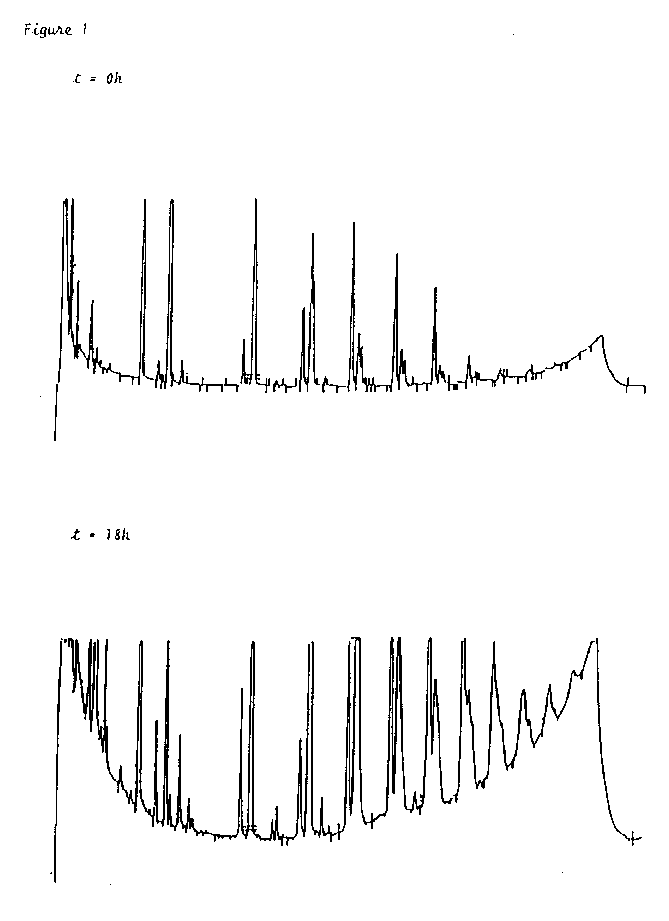

Le mélange obtenu est ensuite soumis à une analyse chromatographique. Le chromatogramme est représenté à la figure 1.

Le mélange de réaction contient à ce moment environ 27 % de glucose, 3 % de fructose et 3 % de saccharose et 67 % de fructanes avec un DP>2, dont environ 60 % ont un DP<12 et 40 % ont un DP$\geqq$, 12. Cette fraction avec un DP$\geqq$, 12 ne peut pas être précipitée avec 80 % d'éthanol, ce qui est une indication de leur nature ramifiée. Environ 40 % des molécules ayant un DP de 3 à 12 sont ramifiées (cfr chromatogramme figure 1).

Les monosaccharides sont principalement éliminés par séparation chromatographique.

Le produit final ainsi obtenu contient environ 90 à 95 % de oligosaccharides avec un degré de polymérisation moyen égal à 4,5-5 et où 40 à 50 % des molécules sont ramifiées.

Les fructo-oligosaccharides ramifiés présentent des propriétés de tolérance inattendues, tout en conservant les autres propriétés avantageuses des fructo-oligosaccharides linéaires : goût neutre et sucré, absence d'odeur, faible valeur calorique, effet de fibres alimentaires, effet bifidogène, caractère naturel et non toxique.

Les fructo-oligosaccharides ramifiés peuvent également être utilisés comme sucre de remplacement dans toutes les applications classiques et industrielles décrites pour les fructo-oligosaccharides linéaires.

La tolérance améliorée de ces fructo-oligoposaccharides ramifiés a été démontrée en réalisant les tests in vitro et in vivo suivants :

1. Tests IN VITRO.

Des matières fécales fraîches de sujets masculins en bonne santé ont été homogénéisées avec 5 fois leur poids de NaKCl isotonique dans un environnement anaérobique. Cinq fractions aliquotes de 10 ml de ce mélange homogénéisé ont été utilisées par groupe de tests.

Comme groupe de tests de référence, on a réalisé 5 incubations dans des éprouvettes contenant 20 g/l d'un mélange de fructo-oligosaccharides linéaires composé de 5 % de glucose, de fructose et de saccharose, de 35 % de GF2, de 50 % de GF3 et 10 % de GF4 (G= glucose, F=Fructose).

Comme deuxième groupe de tests, on a réalisé 5 incubations dans des éprouvettes contenant 20 ml/l du mélange de fructo-oligosaccharides ramifiés décrit à l'exemple 1.

Les éprouvettes sont obturées par une feuille de parafine dont le déplacement indique la production de gaz.

La formation de gaz a été suivie pendant 12 heures. Elle est exprimée en centimètres de déplacement par heure.

Ces tests montrent que la formation de gaz par heure pour les fructo-oligosaccharides ramifiés n'atteint que 35 % de celle due aux fructo-oligosaccharides linéaires.

2. Tests IN VIVO.

Lors d'une expérience effectuée sur trois sujets masculins sensibles à la fermentation dans le gros intestin, différentes quantités de fructo-oligosaccharides ont été mélangées dans du jus de fruits pris au petit-déjeuner, ce petit-déjeuner étant habituellement composé de jus de fruits, de pain et de café en quantité habituelle. La dose introduite est graduellement augmentée par doses de 10 g, à partir d'une dose initiale de 10 g également. Entre deux petits-déjeuners de test, il se passe au moins deux jours sans aucun test.

Les réactions des sujets ont été notées pour chaque concentration, et plus spécifiquement en ce qui concerne les critères suivants : flatulence, bruits gastriques, crampes intestinales, selles molles et diarrhée. La "dose d'arrêt" est définie comme la première dose où un ou plusieurs de ces effets secondaires sont ressentis comme gênants.

Les substances testées qui ont été introduites dans le jus de fruits sont les suivantes :

substance A : mélange de fructo-oligosaccharides linéaires tels que décrits ci-dessus dans l'état de la technique;

substance B : mélange de fructo-oligosaccharides ramifiés tels que décrits dans l'exemple 2.

substance C : mélange contenant 50 % de la substance A et 50 % de la substance B.

Les résultats obtenus sont décrits dans le tableau suivant :

| DOSE D'ARRET | A | B | C |
|---|---|---|---|
| VOLONTAIRE 1 | 20 g | 40 g | 30 g |
| VOLONTAIRE 2 | 30 g | 60 g | 50 g |
| VOLONTAIRE 3 | 40 g | 60 g | 50 g |

D'après ce tableau, on peut conclure que les fructo-oligosaccharides ramifiés sont mieux tolérés que les oligosaccharides linéaires et qu'en introduisant une certaine quantité de fructo-oligosaccharides ramifiés dans un mélange comprenant également des fructo-oligosaccharides linéaires, on augmente la tolérance de ce mélange.

## Propriétés

### Produit faiblement calorique

Les fructo-oligosaccharides ramifiés ne sont pas hydrolysés par les enzymes digestifs de l'homme. Ces substances sont donc d'un apport calorique faible.

### Produit faiblement cariogène

Les fructo-oligosaccharides ramifiés sont moins cariogènes que le saccharose. Ceci s'explique par le fait qu'ils ne sont pas utilisés comme substrat pour la formation de la plaque dentaire et qu'ils sont moins en mesure de causer la formation d'acide par la flore buccale.

### Produits à activité bifidogène et améliorant la flore intestinale

Les fructo-oligosaccharides ramifiés possèdent un effet bifidogène sélectif : ils stimulent de façon spécifique la croissance de la population bifide dans le gros intestin. Ceci s'explique par le fait que ces fructo-oligosaccharides sont utilisés comme source d'énergie par les bactéries appartenant au groupe des bifidobactéries, alors que les autres bactéries utilisent beaucoup plus difficilement ce substrat. En particulier, un certain nombre de bactéries putréfactives (telles que Salmonella et Clostridium) voient leur croissance limitée, ce qui entraîne une amélioraration qualitative et quantitative de la flore intestinale.

### Produit à effet de fibres alimentaires et diminuant le taux de cholestérol.

Les fructo-oligosaccharides possèdent un effet de fibres alimentaires : ils diminuent le temps de transit intestinal et augmentent la masse fécale. Ceci s'explique par le fait que ces fructo-oligosaccharides ne sont pas assimilés dans l'intestin grêle et passent dans le gros intestin où ils subissent une fermentation. Ces fructo-oligosaccharides provoquent également une diminution du taux de cholestérol dans le sang du fait de leur effet de fibres alimentaires.

### Agent diurétique

Les fructo-oligosaccharides ramifiés peuvent être utilisés pour la prévention ou le traitement de tous les troubles qui peuvent être causés par la présence de produits comme par exemple des produits de dégradation accumulés qui peuvent être la cause de troubles aux reins, au foie ou du cancer,...

### Produit améliorant la tolérance des autres produits.

On sait déjà que les fructo-oligosaccharides ramifiés sont mieux tolérés que leurs analogues linéaires.
En effet les molécules ramifiées constituent des substrats de fermentation plus difficiles pour la flore intestinale; la fermentation est donc freinée par la présence de ces molécules.
Il est donc possible d'améliorer la tolérance aux aliments fermentescibles en ajoutant des fructo-oligosaccharides ramifiés à l'alimentation, ceci du fait de la diminution et du ralentissement des phénomènes de fermentation.

### Solubilité du produit.

Les fructo-oligosaccharides ramifiés possèdent une solubilité plus élevée que celle des composés linéaires, ce qui permet une mise en oeuvre plus aisée dans un certain nombre de produits (boissons rafraîchissantes ou à base de lait, autres produits laitiers, confiserie, biscuiterie,...) où de fortes concentrations en sucres doivent être utilisées.
La conjonction de ces deux dernières propriétés, à savoir la tolérance accrue des fructo-oligosaccharides ramifiés et la solubilité accrue du produit selon l'invention favorise leur utilisation comme agent de masse.

Stabilité accrue en mileu acide

Les fructo-oligosaccharides ramifiés possèdent une meilleure stabilité en milieu acide que les composés linéaires, ce qui permet de limiter leur dégradation (formation de saccharides tels que fructose, glucose et saccharose) pendant la fabrication et la conservation d'aliments acides et de garantir à ces derniers des caractéristiques plus constantes pendant leur durée de vie.

L'accroissement de la stabilité en milieu acide a été mis en évidence lors d'une expérience comparative réalisée avec deux substances :

substance A : mélange de fructo-oligosaccharides linéaires contenant

5 % de glucose, fructose et saccharose

35 % de GF2

50 % de GF3

10 % de GF4

substance B : mélange de fructo-oligosaccharides ramifiés tels que décrits dans l'exemple 1.

Chacune des substances A et B a été mise en solution acqueuse à 10° Brix; le pH a ensuite été ajusté à 3,5 à l'aide d'acide chlorhydrique.

Les solutions ainsi obtenues ont été conservées plusieurs semaines à 20°C. La stabilité des fructo-oligosaccharides dans ces solutions a été suivie par l'évolution des teneurs en glucose et saccharose (déterminées par chromatographie gazeuse) en cours de conservation.

Les résultats obtenus sont décrits dans le tableau ci-dessous :

Tableau : Teneur en fructose, glucose et saccharose en %

| Temps de conservation à 20°C et pH = 3,5 | Substance A | Substance B |
|---|---|---|
| O | 5 | 5 |
| 4 semaines | 18 | 11 |
| 8 semaines | 32 | 21 |

## Applications.

Les fructo-oligosaccharides ramifiés peuvent être incorporés dans des aliments ou conditionnés sous forme de granules, ou de tablettes. Dans ce dernier cas ils peuvent être utilisés comme composition pharmaceutique orale, par exemple pour stimuler sélectivement la croissance de la population bifide, comme diurétique, ...

Les fructo-oligosaccharides possèdent des propriétés comparables à celles du sucre et des sirops de glucose; de ce fait on peut les mettre en oeuvre de la même façon. Ils peuvent remplacer le sucre et les sirops de glucose dans la plupart des applications, notamment dans les produits suivants :
- confiserie (bonbons,...),
- produits gélifiés (gommes,...),
- préparations à base de chocolat,
- chewing- gum,
- biscuiterie,
- glaces et sorbets,
- produits laitiers,
- boissons à base de fruits,
- confitures et préparations de fruits,
- caramels,
- préparations pharmaceutiques,...

Les fructo-oligosaccharides ramifiés possèdent en particulier les propriétés adéquates pour remplacer le sucre et les sirops de glucose dans la préparation de produits faiblement caloriques, faiblement cariogènes, bifidogènes, et/ou de régime.

La présente invention est illustrée par une série non limitative d'exemples d'applications possibles.

Comme produit de base, on a utilisé un sirop de fructo-oligosaccharides ramifiés préparé tel que décrit dans l'exemple 1 ou l'exemple 2.

Les produits selon l'invention ont été évalués par un panel d'une vingtaine de dégustateurs afin de comparer leurs propriétés à celles de produits conventionnels à base de saccharose et/ou de sirop de glucose. Ces tests ont révélé une bonne acceptation des produits à base de fructo-oligosaccharides ramifiés.

EXEMPLE 3 : Préparation d'une glace au lait.

**Ingrédients** (en poids) :

|  |  |
|---|---|
| Poudre de lait écrémé | 13,00 |
| Eau | 62,46 |
| Crème (35 % de matière grasse) | 8,60 |
| Fructo-oligosaccharides ramifiés de l'exemple 1 | 15,00 |
| Stabilisateur (Cremodan SE30, Grindsted Products) | 0,50 |
| Arôme vanille (Silesia 111/8309280) | 0,40 |
| Aspartame (NutraSweet) | 0,04 |

Composition :

| | |
|---|---|
| Matière sèche totale | 28,5 % |
| Matière sèche laitière | 16,0 % |
| Matière grasse laitière | 3,0 % |

Préparation :

Les différents ingrédients sous forme de poudre sont mélangés à sec et dissous dans l'eau.

Les fructo-oligosaccharides ramifiés, l'arôme et la crème sont ensuite ajoutés. On mélange jusqu'à l'obtention d'un produit homogène. On chauffe ensuite jusqu'à 80°C et cette température est maintenue pendant 30 secondes. Ensuite, on homogénéisée à chaud (mixer), on refroidit jusqu'à 5°C et on laisse reposer quelques heures au réfrigérateur. On aère (100 %) et on congèle dans une machine à glacer (CARPIGIANI).
On conserve au surgélateur.

EXEMPLE 4 : Préparation d'un sorbet.

**Ingrédients** (en poids) :

|  |  |
|---|---|
| Purée de fraises (6,5 % de matière sèche) | 74,48 |
| Fructo-oligosaccharides ramifiés de l'exemple 1 | 25,00 |
| Stabilisateur (Fructodan SL64, Grinsted Products) | 0,45 |
| Aspartame (NutraSweet) | 0,07 |

Composition : 25 % de matière sèche.

Préparation :

Les Ingrédients sont bien mélangés, chauffés à 80°C et maintenus à cette température pendant 30 secondes. Ensuite, le mélange est refroidi à 5°C et maintenu au réfrigérateur pendant quelques heures. On aère (75 %) et congèle dans une machine à glacer (CARPIGIANI).
On conserve au surgélateur.

EXEMPLE 5 : Préparation d'une confiture.

```
Ingrédients (en poids) :
        Fraises (10 % de matière sèche)            65,00
        Fructo-oligosaccharides ramifiés de        42,00
        l'exemple 1
        Eau                                        25,00
        Pectine (LM 27NH95, Sanofi Bio-Industrie)   1,00
        Acide citrique (50 %)                       0,80
```

Composition : 40 % de matière sèche.

Préparation :

La pectine est dissoute dans de l'eau chaude (60°C), puis elle est bien mélangée avec les fraises. On laisse cuire tout en remuant bien le mélange, jusqu'à l'obtention d'un produit d'un poids de 57 g.
Les fructo-oligosaccharides ramifiés, chauffés à 60°C sont alors ajoutés. On ajoute ensuite l'acide citrique. On laisse refroidir jusqu'à 75°C et on conditionne en pots de verre.

EXEMPLE 6 : Préparation d'un yoghourt.

```
Ingrédients (en poids)
        Lait entier (3,7 % de matière grasse)      94,10
        Poudre de lait écrémé                        1,90
        Ferments de yoghourt                         1,00
        Fructo-oligosaccharides ramifiés de          3,00
        l'exemple 1
```

Composition :

| Matière sèche totale | 16,0 % |
| Matière sèche laitière | 13,5 % |
| Matière grasse laitière | 3,5 % |

Préparation :

La poudre de lait et les fructo-oligosaccharides ramifiés sont mélangés avec le lait froid. Ensuite, on chauffe le mélange jusqu'à 60°C et on l'homogénéise. On le chauffe alors jusqu'à 90°C et on maintient cette température pendant 10 minutes.
On refroidit jusqu'à 45°C et on ensemence avec des ferments lactiques. On transvase le mélange dans des bocaux préchauffés et on incube à 43°C jusqu'à un pH de 4,2.
On conserve à 10°C maximum.

EXEMPLE 7 : Préparation d'un cake.

<u>Ingrédients</u> (en poids).

| | |
|---|---|
| Fructo-oligosaccharides ramifiés de l'exemple 1 | 25,30 |
| Margarine | 8,50 |
| Oeufs entiers | 10,10 |
| Carbonate d'ammonium/bicarbonate | 0,40 |
| Farine | 33,65 |
| Lait | 6,80 |
| Raisins | 10,10 |
| Fruits secs | 5,10 |
| Acésulfame K | 0,05 |

Préparation :

Mélanger les fructo-oligosaccharides ramifiés avec la margarine, ajouter les oeufs, et le carbonate d'ammonium, l'Acésulfame K et le bicarbonate dissous dans 0,5 litre de lait. Ajouter la farine et le reste du lait. Mélanger jusqu'à l'obtention d'un mélange homogène. Ajouter alors les raisins et les fruits secs. Transvaser le tout dans un moule et enfourner à 250°C.

EXEMPLE 8 : Préparation d'une génoise.

<u>Ingrédients</u> (en poids) :

| | |
|---|---|
| Fructo-oligosaccharides ramifiés de l'exemple 1 | 27,00 |
| Oeufs | 49,80 |
| Farine | 27,00 |
| Beurre | 8,30 |
| Acésulfame K (Hoechst) | 0,05 |

Préparation :

Battre les fructo-oligosaccharides ramifiés et les oeufs entiers au bain-marie jusqu'à l'obtention d'une mousse épaisse, d'une densité de +/-0,75. Tamiser la farine et l'ajouter à la pâte, en mélangeant doucement. Chauffer le beurre jusqu'à ce qu'il soit à moitié liquide et le mélanger à la pâte. Verser la pâte dans un moule déjà graissé et cuire au four à 175° pendant 20 minutes.

EXEMPLE 9 : Préparation de gommes.

**Ingrédients** (en poids) :

| | |
|---|---|
| Fructo-oligosaccharides ramifiés de l'exemple 2 | 94,00 |
| Gélatine 200 BLS (Sanofi Bio-Industrie) | 6,50 |
| Eau | 13,50 |
| Solution d'acide citrique à 50 % | 1,50 |
| Cola LE 1613 (Sanofi Bio-Industrie) | 0,10 |
| Aspartame | 0,26 |

Matière sèche du produit fini : 80 %.

Préparation :

La gélatine est dissoute dans de l'eau chaude (80° -90°C). Les fructo-oligosaccharides ramifiés sont cuits à 115°C. Ensuite, le sirop est refroidi jusqu'à 100°C. La solution de gélatine est alors ajoutée à ce sirop. Les bulles d'air sont éliminées de ce sirop en l'amenant sous vide ou en le laissant reposer. La masse est refroidie jusqu'à environ 80°C. L'arôme, le colorant, l'Aspartame et l'acide citrique sont ajoutés et le mélange est coulé dans des formes en amidon. L'amidon est utilisé à une température d'environ 30°-35°C. Les gommes sont recouvertes d'une couche d'amidon et sont gardées au repos pendant 24 heures à température ambiante.
Les gommes sont ensuite dépoudrées et recouvertes d'huile.

EXEMPLE 10 : Préparation de gommes dures.

**Ingrédients** (en poids) :

| | |
|---|---|
| Fructo-oligosaccharides ramifiés de l'exemple 2 | 96,50 |
| Gélatine GAT 15 | 11,00 |
| Eau | 16,50 |
| Solution d'acide citrique à 50 % | 1,50 |
| Arôme mandarine LE 1450 (Sanofi Bio-Industries) | 0,25 |
| Aspartame | 0,28 |

Matière sèche du produit fini : 88 %

Préparation :

La gélatine est dissoute dans de l'eau chaude (80°-90°C). Les fructo-oligosaccharides ramifiés sont cuits à 113°C. Ensuite, le sirop est refroidi jusqu'à 100°C. La solution de gélatine est alors ajoutée à ce sirop. Les bulles d'air sont éliminées de ce sirop, et la masse est refroidie jusqu'à environ 80°C. L'arôme, le colorant, l'Aspartame et l'acide citrique sont ajoutés. Le mélange est coulé dans des formes en amidon. L'amidon est utilisé à une température d'environ 30°-35°C.
Les gommes sont couvertes d'une couche d'amidon.
On les laisse reposer dans un four ventilé à 50°C pendant 72 heures. Ensuite, les gommes sont dépoudrées et huilées.

EXEMPLE 11 : Préparation de sucre cuit (bonbons).

**Ingrédients** (en poids) :

| | |
|---|---|
| Fructo-oligosaccharides ramifiés de l'exemple 1 | 125,00 |
| Acide citrique (100 %) | 0,83 |
| Extrait BE4017 (Sanofi Bio-Industrie) | 0,10 |
| Arôme citron LE 1616 (Sanofi Bio-Industrie) | 0,15 |
| Acésulfame K | 0,34 |

Matière sèche du produit fini : 98 %.

Préparation :

Les fructo-oligosaccharides ramifiés sont chauffés à une température de 165°C, jusqu'à obtention d'une matière sèche de 98 %.
Le sirop est refroidi jusqu'à une température de +/-110°C; ensuite, le colorant, l'arôme, l'Acésulfame K et l'acide citrique sont mélangés au produit obtenu. Le produit obtenu est ensuite versé dans des moules. Après refroidissement les bonbons sont retirés des moules.

EXEMPLE 12 : Préparation de caramels.

**Ingrédients** (en poids) :

| | |
|---|---|
| Fructo-oligosaccharides ramifiés de l'exemple 1 | 68,60 |
| Lait concentré sucré | 26,00 |
| Matière grasse à point de fusion de 32°C | 5,20 |
| Sel | 0,20 |
| Lécithine | 0,20 |
| Acésulfame K | 0,24 |

Préparation :

Dissoudre la lécithine dans la matière grasse au bain-marie à 70°C.
Verser les fructo-oligosaccharides ramifiés, le lait concentré sucré, le sel, l'Acésulfame K et le mélange composé de matière grasse et de lécithine dans un cuiseur. Mélanger et chauffer le mélange jusqu'à 55-60°C.
Augmenter la température jusqu'à 119-121°C. Verser ensuite sur une table de refroidissement et laisser refroidir jusqu'à 35-40°C.

EXEMPLE 13 : Préparation d'un pudding

Ingrédients (en poids) :

| | |
|---|---|
| Lait écrémé | 84,94 |
| Poudre de lait écrémé | 1,80 |
| Fructo-oligosaccharides ramifiés de l'exemple 1 | 10,00 |
| Aspartame (NutraSweet) | 0,03 |
| Amidon (Snowflake 06304, Cerestar) | 3,00 |
| Carraghénane (Genulacta SGI-1, Hercules) | 0,20 |
| Arôme vanille (Flav-o-lok 630019H,PFW Products) | 0,02 |
| Colorant jaune crème (51798,Ned.Kleurstofindustrie) | 0,01 |

Composition : 20 % de matière sèche.

Préparation :

Les ingrédients en poudre sont mélangés à sec; ensuite, on ajoute le lait froid. On mélange alors jusqu'à l'obtention d' un mélange homogène.
On chauffe jusqu'à 95°C et on maintient le mélange à cette température pendant 5 minutes.
On refroidit le tout jusqu'à 50°C en agitant le mélange. On conditionne dans des coupes et on conserve au réfrigérateur.

EXEMPLE 14 : Préparation d'une boisson à base de fruits.

Ingrédients (en poids) :

| | |
|---|---|
| Jus de fruit concentré (Orange PG31332, Quest Int.) | 3,80 |
| Fructo-oligosaccharides ramifiés de l'exemple 1 | 7,00 |
| Saccharose | 7,50 |
| Acide citrique (50 %) | 0,30 |
| Eau | 81,40 |

Composition :

| | |
|---|---|
| Matière sèche | 14,5 % |
| Jus de fruit | 15,0 % |

Préparation:

Dissoudre le sucre dans l'eau. Ajouter les autres ingrédients et mélanger efficacement. Conserver au réfrigérateur.

### Exemple 15: Traitement enzymatique de l'inuline

Une solution de base à 40 Brix, constituée de 50% de saccharose et de 50% d'inuline, contenant une large proportion de molécules à un degré de polymérisation (DP) élevé, est ajustée à un pH de 5,4. Cette solution est divisée en deux fractions.

On ajoute à la première fraction une solution de levansucrase (LS) de telle manière à obtenir 2 U de levansucrase par gramme de matière sèche en portant le pH à 5. La solution est ensuite incubée à 37°C pendant 18 heures. Ensuite la réaction enzymatique est arrêtée par la cuisson du mélange de réaction pendant cinq minutes.

A la seconde fraction on ajoute 0,6 U d'endoinulase, obtenue à partir de la préparation enzymatique Novozyme 230, par gramme de matière sèche. L'incubation est arrêtée après une heure par cuisson du mélange de réaction pendant cinq minutes. Ensuite on ajoute une solution de levansucrase comme décrit plus haut.

Le pourcentage de précipitation dans une solution de 80% d'éthanol est déterminé pour les différentes fractions:

| | % de précipité calculé sur l'ensemble des produits avec DP > 2 |
|---|---|
| solution de base | 66 |
| solution de base + LS | 40 |
| solution de base + endoinul. | 15 |
| solution de base + endoinul. + LS | 2,5 |

Ceci démontre que l'action de la levansucrase a des conséquences importantes sur la solubilité des carbohydrates linéaires du type inuline. Il est évident que d'autres chaînes linéaires, p. e. à base de liaisons 2 → 6 (type phlein) peuvent aussi bien être ramifiées en employant un enzyme adéquat, qui forme préférentiellement d'autres liaisons que celles de la chaîne linéaire, comme par exemple du type 2 → 1 (le fructosyltransférase). Cette technique pourrait même être appliquée à des chaînes glucosidiques.

En partant d'une inuline à un DP moyen nettement inférieur à celle utilisée dans l'exemple, p.e. en partant de l'inuline de topinambour, on peut faire agir directement la levansucrase sur l'inuline sans hydrolyse préalable.

On donne, en forme de tableau, l'analyse chromatographique en phase gazeuse des différentes fractions. Comme on peut le voir dans ce tableau, on constate une augmentation de la proportion de produits à DP 5, DP 6, DP 7, DP 8, DP 9, DP 10 et de produits solubles qui n'ont pas pu être séparés par la colonne employée (DP 10+). Sur le chromatogramme on peut en plus voir que la forme de certains pics du produit de réaction est différente des pics du produit de départ (la présence d'une épaule, indiquant la présence de deux produits) et qu'en plus on a formé d'autres produits qu'on ne peut non plus retrouver dans le chromatogramme du levane. Ceci démontre bien que la levansucrase a transféré les molécules de fructose à partir du sucrose vers la chaîne linéaire.

Ensuite on élimine le glucose de la fraction qui a été traitée avec l'endoinulase et le levansucrase par fermentation. Le produit ainsi obtenu est perméthylé suivant la méthode légèrement adaptée décrite dans l'article de Goran Larson et al.: "Application of a simple methylation procedure for the analysis of glycosphingolipids" paru dans Carbohydrate Research, Vol 161, p. 281-290 (1987).

Après la perméthylation, l'hydrolyse qui permet de produire des monosaccharides, et la silylation des monosaccharides, ceux-ci sont séparés en diméthyl-, triméthyl- et tetraméthylfructoses par chromatographie en phase gazeuse. La présence du diméthylfructose dans le chromatogramme est une preuve de la présence dans les chaînes saccharidiques de molécules de fructose liées à trois autres molécules. En plus on peut détecter des isomères du triméthylfructose comme le 3,4,6 - et le 1,3,4 triméthylfructose indiquant la présence dans les molécules de liaison 1 → 2 et 2 → 6. Ceci prouve bien que l'action de la levansucrase sur la chaîne linéaire de l'inuline donne lieu à des ramifications aussi bien du type:

$$\text{Fru1} \longrightarrow 2\text{Fru1} \longrightarrow 2\text{Fru1} \longrightarrow$$
$$\begin{array}{c} 6 \\ | \\ 2\text{Fru} \end{array}$$

que du type:

$$\text{Fru1} \longrightarrow 2\text{Fru}$$
$$\begin{array}{c} 6 \\ | \\ 2\text{Fru} \end{array}$$

Le tableau I qui suit indique le résultat de l'analyse chromatographique en phase gazeuse des différentes fractions des produits de l'exemple 15.

## TABLEAU I

| | INULINE+SACCHAROSE (50/50) (A) | A+LS | A+ENDO 1hr (B) | B+LS |
|---|---|---|---|---|
| Fructose | 1,3 | 4,8 | 1,4 | 3,6 |
| Glucose | 0,45 | 28 | 0,5 | 26,4 |
| Saccharose | 49,7 | 9,5 | 51,4 | 4,6 |
| DFA | 0,2 | 0,3 | 0,3 | 0,2 |
| F2 | 0,4 | 0,2 | 0,4 | 0,3 |
| GF2 | 1,3 | 0,6 | 1,3 | 0,8 |
| F3 | 1,6 | 0,01 | 6,5 | 2 |
| GF3 | 1,8 | 0,7 | 2 | 1 |
| F4 | 1,0 | 0,1 | 5,5 | 4,5 |
| GF4 | 2 | 0,9 | 4,3 | 1,6 |
| F5 | 0,4 | 2,4 | 3 | 4,9 |
| GF5 | 1,8 | 0,9 | 3,7 | 2,5 |
| F6 | 0,3 | 0,2 | 2 | 3,2 |
| GF6 | 1,4 | 1 | 3 | 2,9 |
| F7 | 0,1 | 0,1 | 1,2 | 2,7 |
| GF7 | 1,2 | 1,1 | 1,2 | 3 |
| F8 | 0,1 | 0,2 | 0,3 | 2,3 |
| GF8 | 1 | 1,3 | 0,6 | 2,8 |
| F9 | 0,05 | 0,1 | 0,2 | } 1,8 |
| DP10 | 0,2 | 1,1 | 0 | |
| DP10+ | 0 | 22 | 3,8 | 27 |
| Précipité | 33 | 23,5 | 7,1 | 1,7 |

(DFA=Di-Fructose Anhydride)

**Revendications**

1. Fructo-oligosaccharide ramifié composé d'une chaîne principale et d'une ou plusieurs chaînes latérales caractérisé en ce que la chaîne principale et la ou les chaînes latérales sont constituées, en majorité d'unités de fructose, la chaîne principale comporte de 2 à 15 unités et au moins une ramification sur une unité de fructose, à l'exclusion des produits naturels: kestose, O-β-D-fructofuranosyl-(2→1)-O-[O-β-D-fructofuranosyl-(2→6)]-β-D-fructofuranosyl-α-D-glucopyranoside, O-β-D-fructofuranosyl- (2→1)-β-D-fructofuranosyl-(2→1)-O-[O-β-D-fructofuranosyl-(2→6)]-β-D-fructofuranosyl-α-D-glucopyranoside et des produits ayant la structure suivante:

```
X ──┌── 2Fru6  -  2 Fru6 ──┐── Y
    │    1                 │
    │    │                 │
    │    2Fru              │
    │                      │
    └                      ┘  n
```

|      |                |         |               |
|------|----------------|---------|---------------|
| a)   | X = Glu1       | n = 2   | Y = 2Fru      |
| b)   | X = Glu1       | n = 3   | Y = 2Fru6-2Fru |
| c)   | X = Fru1       | n = 3   | Y = 2Fru6-2Fru |
| d)   | X = Glu1       | n = 3   | Y = 2Fru      |
| e)   | X = Glu1-2Fru6 | n = 2   |               |
| f)   | X = Glu1       | n = 1   | Y = -(2Fru6)-$_m$ |

$$m = 1 \text{ à } 12$$

2. Fructo-oligosaccharide ramifié selon la revendication 1 caractérisé en ce que la chaîne principale est constituée d'unités de fructose.

3. Fructo-oligosaccharide ramifié selon la revendication 1 ou 2 caractérisé en ce que la ou les chaînes latérales comportent de 1 à 15 unités.

4. Fructo-oligosaccharide ramifié selon l'une quelconque des revendications 1 à 3 caractérisé en ce que la ou les chaînes latérales sont constituées d'unités de fructose.

5. Fructo-oligosaccharide ramifié selon l'une quelconque des revendications 1 à 4 caractérisé en ce que la ou les chaînes latérales ne sont pas ramifiées.

6. Fructo-oligosaccharide ramifié selon l'une quelconque des revendications 1 à 4 caractérisé en ce qu'une ou plusieurs chaînes latérales sont à leur tour ramifiées en des chaînes latérales supplémentaires.

7. Procédé pour la préparation de fructooligosaccharides ramifiés selon l'une quelconque des revendications 1 à 6 caractérisé en ce qu'on obtient lesdits fructo-oligosaccharides par extraction et hydrolyse partielle de fructanes ramifiés ou linéaires, ou des sources végétales contenant lesdits fructanes.

8. Procédé pour la préparation de fructooligosaccharides ramifiés selon l'une quelconque des revendications 1 à 6 caractérisé en ce qu'on obtient lesdits fructo-oligosaccharides ramifiés par synthèse enzymatique, qui implique des saccharides contenant du fructose et qui agit sur au moins un fructo-oligosaccharide utilisé comme substrat en utilisant un enzyme qui forme des liaisons différentes de celles existant dans le fructo-oligosaccharide utilisé comme substrat.

9. Procédé selon la revendication 8 caractérisé en ce qu'on utilise un fructosyltransférase qui forme des liaisons différentes de celles présentes dans la chaîne principale du fructo-oligosaccharide utilisé comme substrat.

10. Procédé pour la préparation de fructooligosaccharides ramifiés selon l'une quelconque des revendications 1 à 6 caractérisé en ce qu'on obtient lesdits fructo-oligosaccharides ramifiés par synthèse enzymatique à partir de saccharides contenant du fructose, en utilisant simultanément ou non plusieurs enzymes formant des liaisons différentes.

11. Procédé pour la préparation de fructooligosaccharides ramifiés caractérisé en ce qu'on obtient lesdits fructo-oligosaccharides par combinaison des procédés des revendications 7 à 10.

12. Procédé pour améliorer la solubilité des fructanes linéaires caractérisé en ce qu'on effectue une réaction de ramification par un procédé selon l'une quelconque des revendications 7 à 11, précédé ou pas, suivant le degré de polymérisation du fructane linéaire, d'une hydrolyse partielle.

13. Procédé pour améliorer la solubilité des fructanes linéaires selon la revendication 12, le fructane linéaire étant l'inuline.

14. Composition caractérisée en ce qu'elle contient avec d'autres ingrédients, un ou plusieurs fructooligosaccharides ramifiés selon l'une quelconque des revendications 1 à 6 et/ou obtenu par le procédé d'une quelconque des revendications 7 à 11.

15. Composition selon la revendication 14 caractérisée en que le ou les ingrédients sont des produits, tels que des protéines, des lipides ou acides gras, des hydrates de carbone, des fibres alimentaires ou des additifs.

16. Composition selon la revendication 14 ou 15 caractérisée en ce que l'on ajoute des édulcorants tels que le sucrose, le glucose, le fructose, les produits d'hydrolyse de l'amidon, la palatinose, les sucres-alcools tels que le sorbitol, le xylitol, l'érythritol, le mannitol, le maltitol, le lactitol, l'isomalt, le leucritol; des agents de masse faiblement caloriques tels que le polydextrose, la cellulose, l'hémicellulose, les fructo- ou autres oligosaccharides; des édulcorants à haut pouvoir sucrant tels que l'aspartame, l'acésulfame, la saccharine, la stévia, le sucralose et autres édulcorants dipeptidiques.

17. Aliment pour êtres humains ou animaux caractérisé en ce qu'il comprend un ou plusieurs fructooligosaccharides ramifiés selon l'une quelconque des revendications 1 à 6 ou obtenu par le procédé selon l'une quelconque des revendications 7 à 11 ou une composition selon l'une quelconque des revendications 14 à 16.

18. Utilisation du produit selon l'une quelconque des revendications 1 à 6 ou de la composition selon l'une quelconque des revendications 14 à 17 comme agent de masse dans des préparations alimentaires.

19. Utilisation du produit selon l'une quelconque des revendications 1 à 6 ou de la composition selon l'une quelconque des revendications 14 à 17 comme édulcorant.

20. Utilisation du produit selon l'une quelconque des revendications 1 à 6 ou de la composition selon l'une quelconque des revendications 14 à 17 comme aliment faiblement calorique.

21. Utilisation du produit selon l'une quelconque des revendications 1 à 6 ou de la composition selon l'une quelconque des revendications 14 à 17 comme aliment faiblement cariogène.

22. Utilisation du produit selon l'une quelconque des revendications 1 à 6 ou de la composition selon l'une quelconque des revendications 14 à 17 comme argent bifidogène.

23. Utilisation du produit selon l'une quelconque des revendications 1 à 6 ou de la composition selon l'une quelconque des revendications 14 à 17 pour la préparation d'une composition pharmaceutique améliorant la flore intestinale.

24. Utilisation du produit selon l'une quelconque des revendications 1 à 6 ou de la composition selon l'une quelconque des revendications 14 à 17 pour la préparation d'une composition pharmaceutique diminuant le taux de cholestérol.

25. Utilisation du produit selon l'une quelconque des revendications 1 à 6 ou de la composition selon l'une quelconque des revendications 14 à 17 pour la préparation d'une composition pharmaceutique diurétique.

26. Composition pharmaceutique caractérisée en ce qu'elle comprend un fructo-oligosaccharide ramifié selon l'une quelconque des revendications précédentes 1 à 6 ou une composition selon l'une quelconque des revendications 14 à 16.

27. Composition pharmaceutique selon la revendication 26 caractérisée en ce qu'elle comprend en outre un support.

## Patentansprüche

1. Verzweigtes Fructo-Oligosaccharid, das aus einer Hauptkette und einer oder mehreren Seitenketten besteht, dadurch gekennzeichnet, daß die Hauptkette und die Seitenkette oder die Seitenketten überwiegend aus Fructose-Einheiten bestehen, und die Hauptkette 2 bis 15 Einheiten und mindestens eine Verzweigung bei einer Fructose-Einheit aufweist, mit Ausnahme der natürlichen Produkte: Kestose, O-β-D-Fructofuranosy1-(2 → 1)-O-[O-β-D-fructofuranosyl-(2→6)]-β-D-fructofuranosyl-α-D-glucopyranosid, O-β-D-Fructofuranosyl-(2→1)-β-D-fructofuranosyl-(2→1)-O-[O-β-D-fructofuranosyl-(2→6)]-β-D-fructofuranosyl-α-D-glucopyranoside, und der Produkte mit der folgenden Struktur:

```
X --┌-- 2Fru6   - 2Fru6 --┐-- Y
    |   1                  |
    |   |                  |
    |   |                  |
    |   2Fru               |
    |                      |
    |                      |
    └                      ┘  n
```

```
a)   X = Glu1        n = 2      Y = 2Fru
b)   X = Glu1        n = 3      Y = 2Fru6-2Fru
c)   X = Fru1        n = 3      Y = 2Fru6-2Fru
d)   X = Glu1        n = 3      Y = 2Fru
e)   X = Glu1-2Fru6  n = 2
f)   X = Glu1        n = 1      Y = -(2Fru6)-m
                                     m = 1 bis 12
```

2. Verzweigtes Fructo-Oligosaccharid gemäß Anspruch 1, dadurch gekennzeichnet, daß die Hauptkette aus Fructose-Einheiten besteht.

3. Verzweigtes Fructo-Oligosaccharid gemäß Anspruch 1 oder 2, dadurch gekennzeichnet, daß die Seitenkette oder die Seitenketten 1 bis 15 Einheiten aufweisen.

4. Verzweigtes Fructo-Oligosaccharid gemäß irgendeinem der Ansprüche 1 bis 3, dadurch gekennzeichnet, daß die Seitenkette oder die Seitenketten aus Fructose-Einheiten bestehen.

5. Verzweigtes Fructo-Oligosaccharid gemäß irgendeinem der Ansprüche 1 bis 4, dadurch gekennzeichnet, daß die Seitenkette oder die Seitenketten nicht verzweigt sind.

6. Verzweigtes Fructo-Oligosaccharid gemäß irgendeinem der Ansprüche 1 bis 4, dadurch gekennzeichnet, daß eine oder mehrere Seitenketten wiederum in weitere Seitenketten verzweigt sind.

7. Verfahren zur Herstellung von verzweigten Fructo-Oligosacchariden gemäß irgendeinem der Ansprüche 1 bis 6, dadurch gekennzeichnet, daß die Fructo-Oligosaccharide durch Extraktion und partielle Hydrolyse von verzweigten oder linearen Fructanen, oder aus den diese Fructane enthaltenden, pflanzlichen Quellen erhalten werden.

8. Verfahren zur Herstellung von verzweigten Fructo-Oligosacchariden gemäß irgendeinem der Ansprüche 1 bis 6, dadurch gekennzeichnet, daß die verzweigten Fructo-Oligosaccharide durch enzymatische Synthese erhalten werden, die Fructose enthaltende Saccharide einschließt, und die auf mindestens ein als Substrat verwendetes Fructo-Oligosaccharid wirkt, wobei ein Enzym verwendet wird, das Bindungen bildet, die verschieden von den Bindungen sind, die in dem als Substrat verwendeten Fructo-Oligosaccharid vorhanden sind.

9. Verfahren gemäß Anspruch 8, dadurch gekennzeichnet, daß eine Fructosyltransferase verwendet wird, die Bindungen bildet, die verschieden von den Bindungen sind, die in der Hauptkette des als Substrat verwendeten Fructo-Oligosaccharids vorhanden sind.

10. Verfahren zur Herstellung von verzweigten Fructo-Oligosacchariden gemäß irgendeinem der Ansprüche 1 bis 6, dadurch gekennzeichnet, daß die verzweigten Fructo-Oligosaccharide durch enzymatische Synthese aus Fructose enthaltenden Sacchariden erhalten werden, wobei gleichzeitig oder nicht gleichzeitig mehrere Enzyme verwendet werden, die verschiedenartige Bindungen bilden.

11. Verfahren zur Herstellung von verzweigten Fructo-Oligosacchariden, dadurch gekennzeichnet, daß die Fructo-Oligosaccharide durch Kombination der Verfahren der Ansprüche 7 bis 10 erhalten werden.

12. Verfahren zum Verbessern der Löslichkeit der linearen Fructane, dadurch gekennzeichnet, daß eine Verzweigungsreaktion nach einem Verfahren gemäß irgendeinem der Ansprüche 7 bis 11 durchgeführt wird, der je nach dem Polymerisationsgrad des linearen Fructans eine partielle Hydrolyse vorausgeht oder nicht vorausgeht.

13. Verfahren zum Verbessern der Löslichkeit der linearen Fructane gemäß Anspruch 12, wobei das linare Fructan Inulin ist.

14. Zusammensetzung, dadurch gekennzeichnet, daß sie außer anderen Bestandteilen ein oder mehrere verzweigte Fructo-Oligosaccharide enthält, die irgendeinem der Ansprüche 1 bis 6 entsprechen und/oder nach dem Verfahren von irgendeinem der Ansprüche 7 bis 11 erhalten wurden.

15. Zusammensetzung gemäß Anspruch 14, dadurch gekennzeichnet, daß der oder die Bestandteile Produkte wie Proteine, Lipide oder Fettsäuren, Kohlehydrate, Nahrungsmittelfasern oder Additive sind.

16. Zusammensetzung gemäß Anspruch 14 oder 15, dadurch gekennzeichnet, daß Süßstoffe, wie Sucrose, Glucose, Fructose, Stärke-Hydrolyseprodukte, Palatinose, Alkohol-Zucker, wie Sorbit, Xylit, Erythrit, Mannit, Maltit, Lactit, Isomalt, Leucrit; schwach kalorienhaltige Massemittel, wie Polydextrose, Cellulose, Hemicellulose, Fructo- oder andere Oligosaccharide; Süßstoffe mit hohem mit hohem Süßvermögen, wie Aspartam, Acesulfam, Saccharin, Stevia, Sucralose und andere dipeptidischen Süßstoffe zugegeben werden.

17. Nahrungsmittel für Menschen oder Tiere, dadurch gekennzeichnet, daß es ein oder mehrere verzweigte Fructo-Oligosaccharide umfaßt, die irgendeinem der Ansprüche 1 bis 6 entsprechen oder nach dem Verfahren gemäß irgendeinem der Ansprüche 7 bis 11 oder einer Zusammensetzung gemäß irgendeinem der Ansprüche 14 bis 16 erhalten wurden.

18. Verwendung des Produkts gemäß irgendeinem der Ansprüche 1 bis 6, oder der Zusammensetzung gemäß irgendeinem der Ansprüche 14 bis 17 als Massemittel bei Nahrungsmittelpräparaten.

19. Verwendung des Produkts gemäß irgendeinem der Ansprüche 1 bis 6, oder der Zusammensetzung gemäß irgendeinem der Ansprüche 14 bis 17 als Süßstoff.

20. Verwendung des Produkts gemäß irgendeinem der Ansprüche 1 bis 6, oder der Zusammensetzung gemäß irgendeinem der Ansprüche 14 bis 17 als schwach kalorienhaltiges Nahrungsmittel.

21. Verwendung des Produkts gemäß irgendeinem der Ansprüche 1 bis 6, oder der Zusammensetzung gemäß irgendeinem der Ansprüche 14 bis 17 als schwach karieserzeugendes Nahrungsmittel.

**22.** Verwendung des Produkts gemäß irgendeinem der Ansprüche 1 bis 6, oder der Zusammensetzung gemäß irgendeinem der Ansprüche 14 bis 17 als bifidogenes Mittel.

**23.** Verwendung des Produkts gemäß irgendeinem der Ansprüche 1 bis 6, oder der Zusammensetzung gemäß irgendeinem der Ansprüche 14 bis 17 zur Herstellung einer pharmazeutischen Zusammensetzung, die die Darmflora verbessert.

**24.** Verwendung des Produkts gemäß irgendeinem der Ansprüche 1 bis 6, oder der Zusammensetzung gemäß irgendeinem der Ansprüche 14 bis 17 zur Herstellung einer pharmazeutischen Zusammensetzung, die den Cholesterinspiegel senkt.

**25.** Verwendung des Produkts gemäß irgendeinem der Ansprüche 1 bis 6, oder der Zusammensetzung gemäß irgendeinem der Ansprüche 14 bis 17 zur Herstellung einer diuretischen pharmazeutischen Zusammensetzung.

**26.** Pharmazeutische Zusammensetzung, dadurch gekennzeichnet, daß sie ein verzweigtes Fructo-Oligosaccharid gemäß irgendeinem der Ansprüche 1 bis 6, oder eine Zusammensetzung gemäß irgendeinem der Ansprüche 14 bis 16 umfaßt.

**27.** Pharmazeutische Zusammensetzung gemäß Anspruch 26, dadurch gekennzeichnet, daß sie außerdem einen Träger umfaßt.

## Claims

**1.** Branched fructo-oligosaccharide consisting of a main chain and of one or more side chains, characterized in that the main chain and the side chain(s) consist mainly of fructose units, the main chain contains from 2 to 15 units and at least one branching point on one fructose unit, with the exception of the natural products: kestose, O-β-D-fructofuranosyl-(2→1)-O-[O-β-D-fructofuranosyl-(2→6) ]-β-D-fructofuranosyl-α-D-glucopyranoside, O-β-D-fructofuranosyl-(2→1)-β-D-fructofuranosyl-(2→1)-O-[O-β-D-fructofuranosyl-(2→6)]-β-D-fructofuranosyl-α-D-glucopyranoside and products having the following structure:

```
X ┌─ 2Fru6    -   2 Fru6 ─┐─ Y
  │  1                    │
  │  │                    │
  │  2Fru                 │
  └                       └  n
a)              X = Glu1        n = 2   Y = 2Fru
b)              X = Glu1        n = 3   Y = 2Fru6-2Fru
c)              X = Fru1        n = 3   Y = 2Fru6-2Fru
d)              X = Glu1        n = 3   Y = 2Fru
e)              X = Glu1-2Fru6  n = 2
f)              X = Glu1        n = 1   Y = -(2Fru6)-m
                                                m = 1 to 12
```

**2.** Branched fructo-oligosaccharide according to Claim 1, characterized in that the main chain consists of fructose units.

**3.** Branched fructo-oligosaccharide according to Claim 1 or 2, characterized in that the side chain(s) consist(s) of 1 to 15 units.

**4.** Branched fructo-oligosaccharide according to any one of Claims 1 to 3, characterized in that the side chain(s) consist(s) of fructose units.

5. Branched fructo-oligosaccharide according to any one of Claims 1 to 4, characterized in that the side chain(s) is/are unbranched.

6. Branched fructo-oligosaccharide according to any one of Claims 1 to 4, characterized in that one or more side chains are, in their turn, branched into additional side chains.

7. Process for the preparation of branched fructooligosaccharides according to any one of Claims 1 to 6, characterized in that the said fructo-oligosaccharides are obtained by extraction and partial hydrolysis of branched or linear fructans, or from vegetable sources containing the said fructans.

8. Process for the preparation of branched fructooligosaccharides according to any one of Claims 1 to 6, characterized in that the said branched fructooligosaccharides are obtained by enzymatic synthesis, which involves saccharides containing fructose and which acts on at least one fructo-oligosaccharide used as substrate by using an enzyme which forms bonds other than those which exist in the fructo-oligosaccharide used as a substrate.

9. Process according to Claim 8, characterized in that a fructosyltransferase is used which forms bonds other than those present in the main chain of the fructooligosaccharide used as substrate.

10. Process for the preparation of branched fructooligosaccharides according to any one of Claims 1 to 6, characterized in that the said branched fructooligosaccharides are obtained by enzymatic synthesis from saccharides containing fructose by using, optionally simultaneously, several enzymes which form different bonds.

11. Process for the preparation of branched fructooligosaccharides, characterized in that the said fructooligosaccharides are obtained by a combination of the processes of Claims 7 to 10.

12. Process for improving the solubility of linear fructans, characterized in that a branching reaction is carried out by means of a process according to any one of Claims 7 to 11, optionally preceded, according to the degree of polymerization of the linear fructan, by partial hydrolysis.

13. Process for improving the solubility of linear fructans according to Claim 12, the linear fructan being inulin.

14. Composition, characterized in that it contains, with other ingredients, one or more branched fructooligosaccharides according to any one of Claims 1 to 6 and/or obtained by the process of any one of Claims 7 to 11.

15. Composition according to Claim 14, characterized in that the ingredient(s) are products such as proteins, lipids or fatty acids, carbohydrates, dietary fibers or additives.

16. Composition according to Claim 14 or 15, characterized in that sweeteners such as sucrose, glucose, fructose, hydrolysis products of starch, palatinose, the sugar alcohols such as sorbitol, xylitol, erythritol, mannitol, maltitol, lactitol, isomalt or leucritol; low-calorie bulking agents such as polydextrose, cellulose, hemicellulose or the fructo- or other oligosaccharides; sweeteners with a high sweetening power such as aspartame, acesulfame, saccharin, stevia, sucralose and other dipeptide sweeteners are added.

17. Food for human beings or animals, characterized in that it comprises one or more branched fructooligosaccharides according to any one of Claims 1 to 6 or obtained by the process according to any one of Claims 7 to 11 or a composition according to any one of claims 14 to 16

18. Use of the product according to any one of Claims 1 to 6 or of the composition according to any one of Claims 14 to 17 as a bulking agent.

19. Use of the product according to any one of Claims 1 to 6 or of the composition according to any one of Claims 14 to 17 as a sweetener.

20. Use of the product according to any one of Claims 1 to 6 or of the composition according to any one of Claims 14 to 17 as a low-calorie food.

21. Use of the product according to any one of Claims 1 to 6 or of the composition according to any one of Claims 14 to 17 as a weakly cariogenic food.

22. Use of the product according to any one of Claims 1 to 6 or of the composition according to any one of Claims 14 to 17 as a bifidogenic agent.

23. Use of the product according to any one of Claims 1 to 6 or of the composition according to any one of Claims 14 to 17 as an agent for improving the intestinal flora.

24. Use of the product according to any one of Claims 1 to 6 or of the composition according to any one of Claims 14 to 17 as an agent for reducing the cholesterol level.

25. Use of the product according to any one of Claims 1 to 6 or of the composition according to any one of Claims 14 to 17 as an agent with a dietary fiber effect.

26. Pharmaceutical composition, characterized in that it comprises a branched fructo-oligosaccharide according to any one of the preceding Claims 1 to 6 or a composition according to any one of Claims 14 to 16.

27. Pharmaceutical composition according to Claim 26 characterized in that it additionally comprises a filler.

Figure 1

$t = 0h$

$t = 18h$